# EUROPEAN PATENT APPLICATION

(11) **EP 4 273 229 A1**
(43) Date of publication of application: **08.11.2023**
(21) Application number: 21915778.1
(22) Date of filing: 28.12.2021
(51) Int. Cl.: C12N 5/00

(54) **METHOD FOR ISOLATING EXOSOMES WITH HIGH EFFICIENCY AND HIGH PURITY**

(30) Priority: 29.12.2020 KR 20200185554
(71) Applicant: Union Korea Life Sciences Inc., Uiwang-si, Gyeonggi-do 16006 (KR)
(72) Inventor: KIM, Jung, Seok, Uijeongbu-si, Gyeonggi-do 11631 (KR); BACK, Bying, Ha, Seongnam-si, Gyeonggi-do 13596 (KR)
(74) Representative: ZBM Patents - Zea, Barlocci & Markvardsen
(86) International application number: PCT/KR2021/020063
(87) International publication number: WO 2022/145980

(57) **Abstract**

The present disclosure relates to an exosome isolation method, more specifically, a method of isolating exosomes with high efficiency and high purity, including deproteinization, exosome aggregation, exosome binding, and exosome isolation. The method of isolating exosomes according to the present disclosure may be applied to all samples, such as body fluids and cell culture fluids, and thus, is characterized as a technique applicable to samples universally, and since total time required for exosome isolation is within 40 minutes, the method is time efficient, and since the method is capable of isolating 25 times more exosomes or greater than ultracentrifugation, the method may isolate exosomes with high efficiency and high purity. Therefore, the isolation method of the present disclosure and the exosomes isolated by the method are expected to be widely applicable in research on diagnosis or treatment methods requiring high-purity exosomes.

## Description

### Technical Field

The present disclosure relates to an exosome isolation method, more specifically, a method of isolating exosomes with high efficiency and high purity, including deproteinization, exosome aggregation, exosome binding, and exosome isolation.

### Background Art

Extracellular vesicle refers to exosomes, ectosomes, micro-vesicles, and apoptotic bodies released or secreted from cells, among which exosomes are biological nanoparticles that have a size of 30 nm to 300 nm, and are generated from multi vesicular bodies (MVB) within cells. Although exosomes are small in size, the exosomes play an important role in physiological responses (for example, immune responses, neural functions, and stem cell maintenance) and disease pathology (for example, cancer, diseases of the liver and heart, degenerative diseases, etc.).

Extracellular vesicles transport various substances, including proteins and RNA, while being secreted and absorbed during intercellular exchange processes. When extracellular vesicles fail to carry out these intracellular exchange processes, various diseases may be caused, and thus, attention is being focused on extracellular vesicles. Extracellular vesicles may be isolated relatively easily from various biofluids such as blood, cerebrospinal fluid, urine, amniotic fluid, breast milk, saliva, and semen, and include various nucleotides or marker proteins depending on the cell or organelle from which the vesicles are derived. For example, oncosomes, which are extracellular vesicles derived from cancer cells, include mRNA of a gene that induces the growth of cancer cells, and extracellular vesicles derived from antigen-presenting cells include major histocompatibility complexes. As such, extracellular vesicles include biological substances such as cell-specific proteins and nucleotides at high concentrations, and thus, the extracellular vesicles have an advantage in that even proteins included in an amount of about 0.01 % of total proteins in general biological fluids, and nucleotides that are hard to detect with common detection methods, may be relatively easily detected in extracellular vesicles. In addition, although the types of proteins or nucleotides present in extracellular vesicles are only a very small part of the total, materials in extracellular vesicles may exhibit unique characteristics of the cells from which they are derived, and therefore, exosome analysis is very useful for the purpose of diagnosing specific diseases, and research on this has been actively conducted recently.

Obtaining high-purity exosomes is important in research on methods of diagnosis or treatment by using exosomes. Regarding the isolation of exosomes, various methods have been studied, including Korean Patent Publication No. 10-2016-0115988. Examples thereof include ultracentrifuge, density centrifuge, use of columns, PEG precipitation, chromatography, immuno-magnetic separation (IMS), and acoustic separation (acoustic purification). Among the methods, exosome isolation methods using ultracentrifugation have been the most used methods to date, but these require special equipment and take a lot of time, while the obtainable yield of extracellular vesicles (EV) is low and the obtained EVs are often damaged, and thus, there is a need for a method capable of improving the yield and isolating high-purity exosomes while improving these problems.

### Disclosure

### Technical Problem

Due to the above needs, the present inventors have established a method of isolating exosomes from biological samples, including deproteinization, exosome aggregation, exosome binding, and exosome isolation, and confirmed that exosomes isolated by the isolation method are isolated with high efficiency and high purity, and completed the present disclosure.

Accordingly, an object of the present disclosure is to provide a method of isolating exosomes with high efficiency and high purity, including:
(a) performing deproteinization on a biological sample;
(b) aggregating exosomes by treating the deproteinized sample with a buffer;
(c) binding the aggregated exosomes to a membrane made of a protein-friendly material or a positive charge-friendly material;
(d) centrifuging and washing the exosomes bound to the membrane; and
(e) isolating the washed exosomes through a neutralization reaction.

In addition, an object of the present disclosure is to provide a buffer composition for isolating exosomes from a biological sample, including at least one selected from the group consisting of polyethylene glycol (PEG), polyetherimide (PEI), and dextran.

In addition, an object of the present disclosure is to provide a kit for isolating exosomes from a biological sample, including a buffer including at least one selected from the group consisting of PEG, PEl, and dextran.

However, technical problems to be solved by the present disclosure is not limited to the above-mentioned problems, and other problems not mentioned will be clearly understood by those skilled in the art from the following description.

### Technical Solution

In order to achieve the above object, an aspect of the present disclosure provides a method of isolating exosomes from a biological sample, including:
(a) performing deproteinization on a biological sample;
(b) aggregating exosomes by treating the deproteinized sample with a buffer;
(c) binding the aggregated exosomes to a membrane made of a protein-friendly material or a positive charge-friendly material;
(d) centrifuging and washing the exosomes bound to the membrane; and
(e) isolating the washed exosomes through a neutralization reaction.

In an embodiment of the present disclosure, the deproteinization of (a) may be performed in a syringe filter having a pore size of 0.2 µm to 1.0 µm.

In another embodiment of the present disclosure, the buffer used in (b) may include at least one selected from the group consisting of polyethylene glycol (PEG), polyetherimide (PEI), and dextran.

In still another embodiment of the present disclosure, in the buffer used in (b), a concentration of PEG may be 0.5 %(w/v) to 30 % (w/v).

In still another embodiment of the present disclosure, in the buffer used in (b), a concentration of PEI may be 0.01 %(w/v) to 10 % (w/v).

In still another embodiment of the present disclosure, in the buffer used in (b), a concentration of dextran may be 0.01 %(w/v) to 10 % (w/v).

In still another embodiment of the present disclosure, the buffer used in (b) may have a pH of 3.5 to 6.5.

In still another embodiment of the present disclosure, a protein-friendly material or a positive charge-friendly material in (c) may be at least one selected from the group consisting of polycarbonate, anodic aluminum oxide (AAO), cellulose membrane, and regenerative cellulose.

In another embodiment of the present disclosure, the neutralization reaction of (e) may be performed by treating a buffer including at least one selected from the group consisting of urea, monosodium phosphate (NaH₂PO₄), and Tris hydrochloride (Tris-HCl).

In still another embodiment of the present disclosure, the buffer used in (e) may have a pH of 7.0 to 9.0.

Another aspect provides a buffer composition for isolating exosomes from a biological sample, including at least one selected from the group consisting of PEG, PEl, and dextran.

Still another aspect provides a kit for isolating exosomes from a biological sample, including a buffer including at least one selected from the group consisting of PEG, PEl, and dextran.

### Advantageous Effects

A method of isolating exosomes according to the present disclosure may be applied to all samples, such as body fluids and cell culture fluids, and thus, the method is a technique applicable to samples universally; since total time required for exosome isolation is within 40 minutes, the method is time efficient; and since the method is capable of isolating 25 times or more exosomes than ultracentrifugation, the method may isolate exosomes with high efficiency and high purity. Therefore, the isolation method of the present disclosure and exosomes isolated by the method are expected to be widely applicable in research on diagnosis or treatment methods requiring high-purity exosomes.

### Description of Drawings

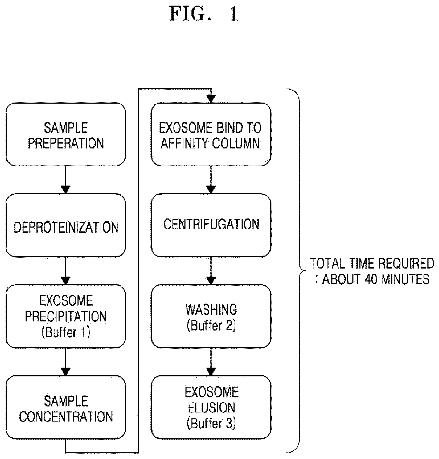

FIG. 1 shows a schematic diagram of a method of isolating exosomes according to the present disclosure.

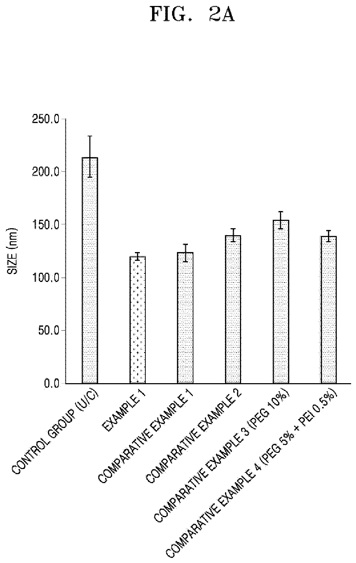

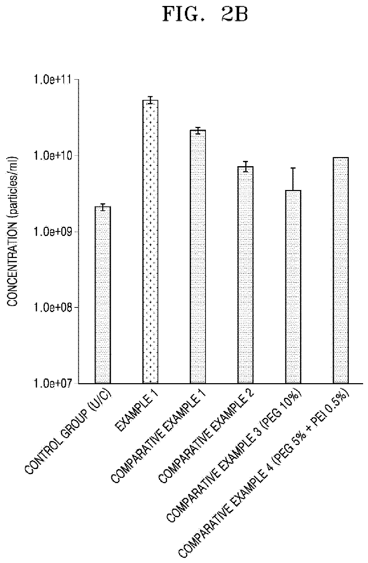

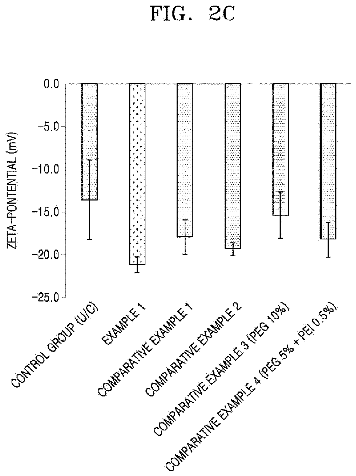

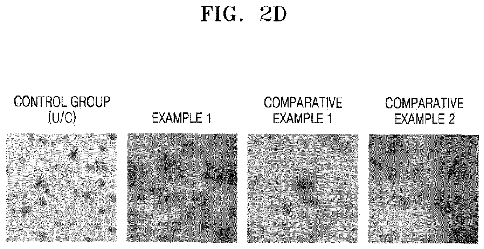

FIG. 2 shows results of comparing exosomes isolated from a human urine sample by an exosome isolation method according to the present disclosure (Example 1), and exosomes isolated in the same manner as in Example 1 except that a buffer of a different composition is used instead of Buffer 1 in aggregation of exosomes (Comparative Example 1: uses a buffer composition of an existing commercially available PEI-based exosome isolation kit (System Biosciences), Comparative Example 2: uses a buffer composition of an existing commercially available PEG+PEI-based exosome isolation kit (Invitrogen), Comparative Example 3: uses a PEG-based buffer, and Comparative Example 4: uses a PEG+PEI-based buffer), in which FIG. 2A compares sizes, FIG. 2B compares particle numbers, FIG. 2C compares zeta-potentials, and FIG. 2D compares electron microscopy images.

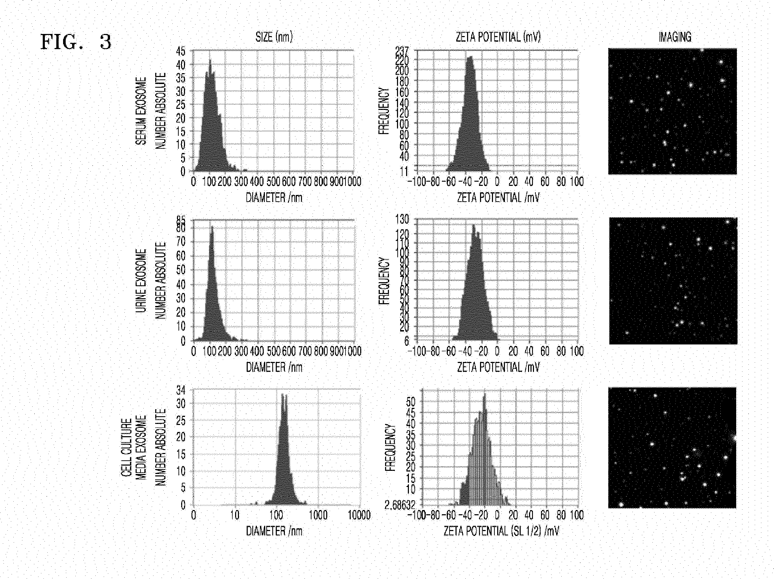

FIG. 3 shows results of exosome isolation for each sample, such as human serum, urine, and cell culture medium.

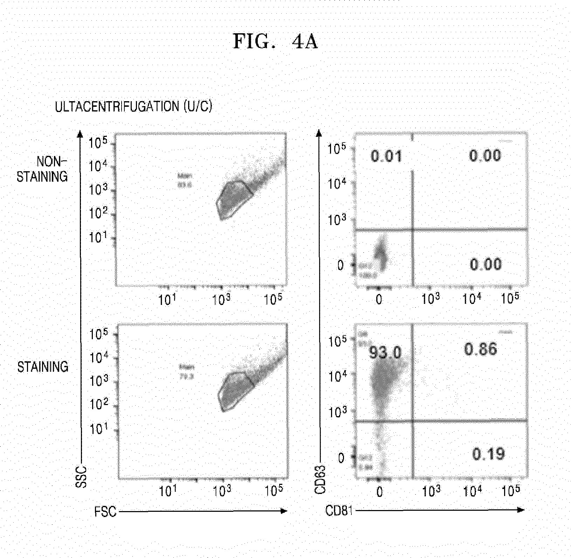

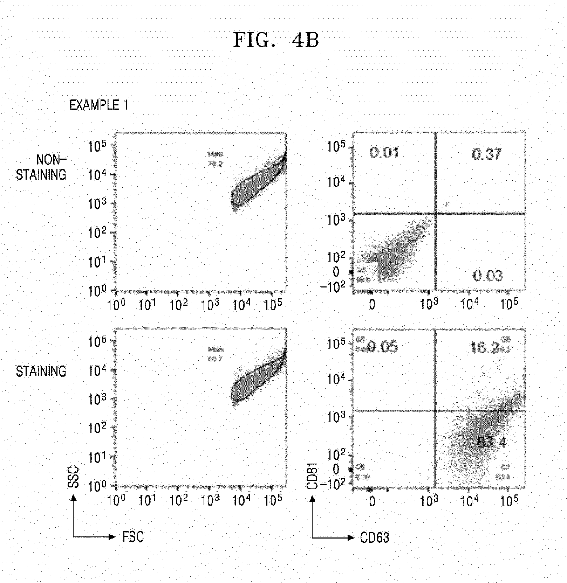

FIG. 4 shows results of analyzing phenotypes of exosomes isolated from a cell culture medium through flow cytometry, in which FIG. 4A shows phenotypes of exosomes (control group) isolated by ultracentrifugation (U/C), and FIG. 4B shows phenotypes of exosomes (Example 1) isolated by a method of the present disclosure.

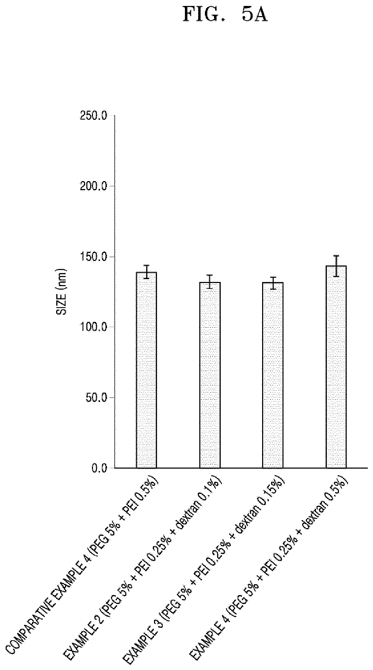

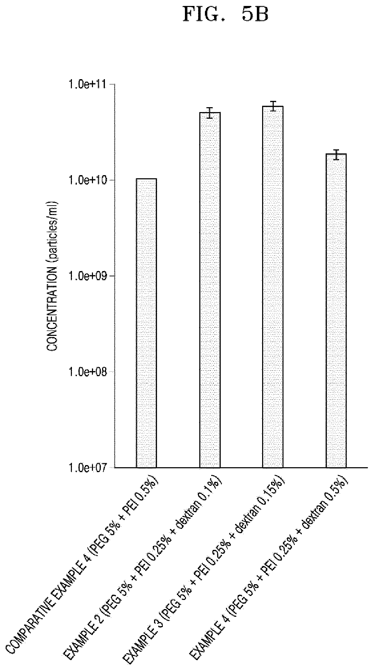

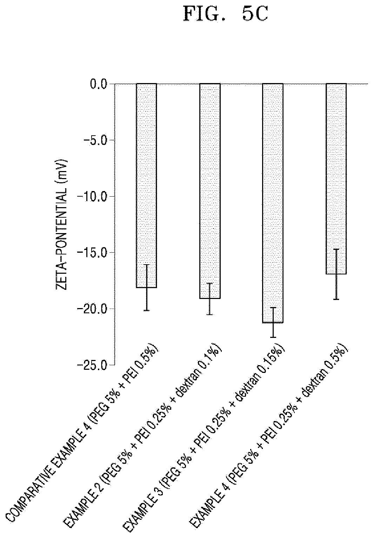

FIG. 5 shows results of comparing exosome isolation efficiencies for each concentration of a PEG+PEI+dextran-based buffer in an exosome aggregation, when isolating exosomes by a method according to the present disclosure from a human urine sample, along with the exosome isolation efficiency of Comparative 4 (PEG+PEI-based buffer), in which FIG. 5A compares sizes, FIG. 5B compares particle numbers, and FIG. 5C compares zeta-potentials.

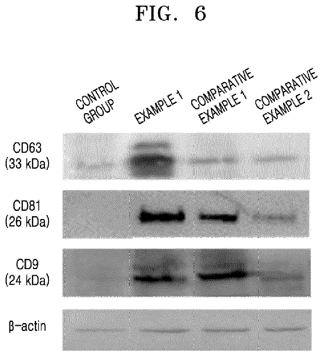

FIG. 6 shows results of performing a Western blot, which is a protein analysis experiment, to confirm protein markers (CD63, CD81, CD9) in: exosomes isolated from human mesenchymal stem cell (MSC) culture media by ultracentrifugation(U/C) (control group); exosomes isolated by an exosome isolation method according to the present disclosure(Example 1); and exosomes isolated in the same manner as in Example 1 except that a buffer of an existing commercially available exosome isolation kit is used instead of Buffer 1 in the aggregation of exosomes (Comparative Example 1: uses a buffer composition of an existing commercially available PEI-based exosome isolation kit (System Biosciences), Comparative Example 2: uses a buffer composition of an existing commercially available PEG+PEI-based exosome isolation kit (Invitrogen)).

### Mode for Invention

Hereinafter, the present disclosure will be described in more detail.

The present inventors established a method of isolating exosomes, including deproteinization, exosome aggregation, exosome binding, and exosome isolation, and confirmed that the exosomes isolated by the isolation method were isolated with high efficiency and high purity, and therefore, completed the present disclosure.

Accordingly, the present disclosure provides a method of isolating exosomes with high efficiency and high purity, including (a) performing deproteinization on a biological sample; (b) aggregating exosomes by treating the deproteinized sample with a buffer; (c) binding the aggregated exosomes to a membrane made of a protein-friendly material or a positive charge-friendly material; (d) centrifuging and washing the exosomes bound to the membrane; and (e) isolating the washed exosomes through a neutralization reaction.

The term, "exosome", used herein, refers to a vesicle secreted from a cell and released into the extracellular space, and may refer to a membrane-structured vesicle, in which the inside and the outside are separated, has plasma membrane lipids, plasma membrane proteins, and nucleic acids, and is a nano-sized vesicle smaller than the original cell. The exosome may bind to other cells to deliver membrane components, mRNAs, miRNAs, etc., and may act as an extracellular transporter that mediates cell-cell communication by delivering these transferred materials to recipient cells.

The exosomes isolated by the above method may have a diameter of 100 nm to 200 nm, for example, 100 nm to 180 nm, 100 nm to 150 nm, 100 nm to 140 nm, 100 nm to 130 nm, 100 nm to 120 nm, 100 nm to 110 nm, 110 nm to 200 nm, 110 nm to 150 nm, 110 nm to 140 nm, 110 nm to 130 nm, or 110 nm to 120 nm.

The term, "exosome isolation method", used herein, refers to a method of isolating exosomes included in a sample, and refers to all methods of isolating and purifying a small amount of exosomes secreted from cells for wide application in treatment or diagnosis.

The term, "high efficiency", used herein, refers to that exosome isolation exhibits excellent production efficiency with high accuracy in a short time, and a highly efficient exosome isolation method in the present disclosure refers to that exosomes are isolated with a high yield.

The term "high purity", used herein, refers to that in exosome isolation, contents of other impurities is lowered and the isolation of exosomes is excellent.

The term "biological sample", used herein, refers to a specimen that may be analyzed biologically, and in the present disclosure, a biological sample may be a tissue, blood, serum, plasma, nasal mucus, saliva, urine, sputum, and lymph fluid isolated from an individual, or cell culture medium, preferably serum, urine, or cell culture medium, but is not limited thereto. The subject may be a mammal, such as a human, a cow, a horse, a pig, a dog, sheep, a goat, or a cat.

Hereinafter, the exosome isolation method of the present disclosure will be subdivided into each process and described in detail.

### [step (a): Deproteinization]

The deproteinization is removing high molecular weight or aggregated proteins in the sample, in the present disclosure, the deproteinization may be performed in a syringe filter with a pore size of 0.2 µm to 1.0 µm, but is limited thereto.

The pore size of the syringe filter may be, for example, 0.2 µm to 0.9 µm, 0.2 µm to 0.8 µm, 0.4 µm to 1.0 µm, 0.4 µm to 0.9 µm, 0.4 µm to 0.8 µm, 0.5 µm to 1.0 µm, 0.5 µm to 0.9 µm, or 0.5 µm to 0.8 µm. When the pore size is too small beyond the above range, inflow of the desired exosomes may be blocked, and when the pore size is too large beyond the above range, deproteinization may not be performed smoothly, resulting in lower exosome purity and a yield.

In an embodiment, the syringe filter of (a) may be a membrane composed of cellulose, polyvinylidene fluoride (PVDF), mixed cellulose esters (MCE), but is not limited thereto, and all syringe filters made of materials commonly used for deproteinization in the art may be included.

### [step (b): Exosome aggregation (exosome precipitation)]

The exosome aggregation is aggregating exosomes by using a buffer and imparting an electric charge to the surface of the exosomes, and a buffer of (b) may include at least one selected from the group consisting of PEG, PEl, and dextran, specifically, may be prepared by mixing at least one selected from the group consisting of PEG, PEl, and dextran in a sodium chloride solution, or phosphate-buffered saline (PBS) solution. The dextran may be, for example, dextran sulfate, or dextran acetate, but is not limited thereto.

In an embodiment, the PEG may have a molecular weight of 100 Da to 10,000 Da, for example, 100 Da to 9,000 Da, 100 Da to 8,000 Da, 100 Da to 7,000 Da, 100 Da to 6,000 Da, 1,000 Da to 10,000 Da, 1,000 Da to 9,000 Da, 1,000 Da to 8,000 Da, 1,000 Da to 7,000 Da, 0,000 Da to 6,000 Da 2,000 Da to 9,000 Da, 2,000 Da to 8,000 Da, 2,000 Da to 7,000 Da, 2,000 Da to 6,000 Da, 3,000 Da to 10,000 Da, 3,000 Da to 9,000 Da, 3,000 Da to 8,000 Da, 3,000 Da to 7,000 Da, 3,000 Da to 6,000 Da, 4,000 Da to 10,000 Da, 4,000 Da to 9,000 Da, 4,000 Da to 8,000 Da, 4,000 Da to 7,000 Da, 4,000 Da to 6,000 Da, 5,000 Da to 10,000 Da, 5,000 Da to 9,000 Da, 5,000 Da to 8,000 Da, 5,000 Da to 7,000 Da, 5,000 Da to 6,000 Da. When the molecular weight is too small beyond the above range, exosome cohesiveness may be reduced, and when the molecular weight is too large beyond the above range, exosomes may over-aggregate, resulting in precipitation.

In an embodiment, a concentration of PEG in the buffer may be 0.5 %(w/v) to 30 %(w/v), such as 0.5 %(w/v) to 25 %(w/v), 0.5 %(w/v) to 20 %(w/v) ), 0.5 %(w/v) to 15 %(w/v), 0.5 %(w/v) to 10 %(w/v), 1.0 %(w/v) to 30 %(w/v), 1.0 %(w/v) to 25 %(w/v), 1.0 %(w/v) to 20 %(w/v), 1.0 %(w/v) to 15 %(w/v), 1.0 %(w/v) to 10 %(w/v), 3.0 %(w/v) to 30 %(w/v), 3.0 %(w/v) to 25 %(w/v), 3.0 %(w/v) /v) to 20 %(w/v), 3.0 %(w/v) to 15 %(w/v), 3.0 %(w/v) to 10 %(w/v), 5.0 %(w/v) ) to 30 %(w/v), 5.0 %(w/v) to 25 %(w/v), 5.0 %(w/v) to 20 %(w/v), 5.0 %(w/v) to 15 %(w/v), or 5.0 %(w/v) to 10 %(w/v).

In an embodiment, a concentration of PEI in the buffer may be 0.01 %(w/v) to 10 %(w/v), for example, 0.01 %(w/v) to 5 %(w/v), 0.01 %(w/v) to 3 %(w/v) ), 0.01 %(w/v) to 1 %(w/v), 0.01 %(w/v) to 0.5 %(w/v), 0.01 %(w/v) to 0.4 %(w/v), 0.05 %(w/v) to 10 %(w/v), 0.05 %(w/v) to 5 %(w/v), 0.05 %(w/v) to 3 %(w/v), 0.05 %(w/v) to 1 %(w/v), 0.05 %(w/v) to 0.5 %(w/v), 0.05 %(w/v) to 0.4 %(w/v), 0.1 %(w/v) /v) to 10 %(w/v), 0.1 %(w/v) to 5 %(w/v), 0.1 %(w/v) to 3 %(w/v), 0.1 %(w/v) ) to 1 %(w/v), 0.1 %(w/v) to 0.5 %(w/v), 0.1 %(w/v) to 0.4 %(w/v), 0.2 %(w/v) to 10 %(w/v), 0.2 %(w/v) to 5 %(w/v), 0.2 %(w/v) to 3 %(w/v), 0.2 %(w/v) to 1 %(w/v), 0.2 %(w/v) to 0.5 %(w/v), or 0.2 %(w/v) to 0.4 %(w/v).

In an embodiment, a concentration of dextran in the buffer may be 0.01 %(w/v) to 10 %(w/v), for example, 0.01 %(w/v) to 5 %(w/v), 0.01 %(w/v) to 3 %(w/v) ), 0.01 %(w/v) to 1 %(w/v), 0.01 %(w/v) to 0.5 %(w/v), 0.01 %(w/v) to 0.4 %(w/v), 0.05 %(w/v) to 10 %(w/v), 0.05 %(w/v) to 5 %(w/v), 0.05 %(w/v) to 3 %(w/v), 0.05 %(w/v) to 1 %(w/v), 0.05 %(w/v) to 0.5 %(w/v), 0.05 %(w/v) to 0.4 %(w/v), 0.1 %(w/v) /v) to 10 %(w/v), 0.1 %(w/v) to 5 %(w/v), 0.1 %(w/v) to 3 %(w/v), 0.1 %(w/v) ) to 1 %(w/v), 0.1 %(w/v) to 0.5 %(w/v), 0.1 %(w/v) to 0.4 %(w/v), 0.2 %(w/v) to 10 %(w/v), 0.2 %(w/v) to 5 %(w/v), 0.2 %(w/v) to 3 %(w/v), 0.2 %(w/v) to 1 %(w/v), 0.2 %(w/v) to 0.5 %(w/v), or 0.2 %(w/v) to 0.4 %(w/v).

When a concentration of the buffer in (b) is too high or too low beyond the above ranges, a yield or purity of the exosome isolation may decrease.

In an embodiment, the buffer used in (b) may have a pH of 3.5 to 6.5, but is not limited thereto, and may be, for example, pH 3.5 to pH 6.0, pH 3.5 to pH 5.6, pH 4.5 to pH 6.5, pH 4.5 to pH 6.0, pH 4.5 to pH 5.6, pH 5.0 to pH 6.5, or pH 5.0 to pH 6.0.

### [step (c): Exosome binding (affinity membrane column)]

Exosome binding is forming a membrane capable of specifically binding to exosomes, and may be performed at room temperature for 1 minute to 5 minutes by using a protein-friendly material or a positive charge-friendly material, but is not limited thereto.

In an embodiment, the protein-friendly material or the positive charge-friendly material may be any one selected from the group consisting of polycarbonate, anodic aluminum oxide (AAO), cellulose membrane, regenerative cellulose, and may be preferably polycarbonate, or regenerative cellulose, but is not limited thereto, and may include all materials capable of specifically binding to exosomes, and commonly used in the related art.

### [step (d): Centrifugation and washing]

In the process, the exosomes bound to the membrane are subjected to a first centrifugation, washed with saline or PBS at a concentration of 2X to 5X, and the washed exosomes are concentrated by a second centrifugation, wherein the first centrifugation may be performed at 300 wg to 800 xg, for example, 300 wg to 700 xg, 300 wg to 600 xg, 300 wg to 500 xg, 400 wg to 800 xg, 400 wg to 700 xg, 400 wg to 600 xg, 400 wg to 500 xg, 500 wg to 800 xg, 500 wg to 700 xg, or 500 wg to 600 xg, at room temperature or 4 °C for 1 minute to 5 minutes, and the second centrifugation may be performed at 2,000 wg to 5,000 xg, for example, 2,000 wg to 4,000 xg, 2,000 wg to 3,000 xg, 2,000 wg to 2,500 xg, 2,200 wg to 5,000 xg, 2,200 wg to 4,000 xg, 2,200 wg to 3,000 xg, or 2,200 wg to 2,200 xg, at room temperature or 4 °C for 5 minutes to 20 minutes.

### [step (e): Exosome isolation (exosome elusion)]

The exosome isolation is neutralizing the buffer used in (b) exosome aggregation to isolate exosomes, wherein the neutralization may be performed by treating a buffer including at least one selected from the group consisting of urea, monosodium phosphate (NaH₂PO₄), and Tris hydrochloride (Tris-HCl), specifically, the buffer may be prepared by mixing at least one selected from the group consisting of urea, NaH₂PO₄, and Tris-HCl into PBS or saline solution.

In an embodiment, a concentration of the urea in the buffer of (e) may be 0.01 M to 10 M, for example, 0.01 M to 5 M, 0.01 M to 3 M, 0.01 M to 1 M, 0.01 M to 0.7 M, 0.01 M to 0.5 M, 0.1 M to 10 M, 0.1 M to 5 M, 0.1 M to 3 M, 0.1 M to 1 M, 0.1 M to 0.7 M, 0.1 M to 0.5 M, 0.2 M to 10 M, 0.2 M to 5 M, 0.2 M to 3 M, 0.2 M to 1 M, 0.2 M to 0.7 M, or 0.2 M to 0.5 M.

In an embodiment, a concentration of NaH₂PO₄ in the buffer of (e) may be 0.01 M to 10 M, for example, 0.01 M to 5 M, 0.01 M to 3 M, 0.01 M to 1 M, 0.01 M to 0.7 M, 0.01 M to 0.5 M, 0.05 M to 10 M, 0.05 M to 5 M, 0.05 M to 3 M, 0.05 M to 1 M, 0.05 M to 0.7 M, 0.05 M to 0.5 M, 0.1 M to 10 M, 0.1 M to 5 M, 0.1 M to 3 M, 0.1 M to 1 M, 0.1 M to 0.7 M, 0.1 M to 0.5 M, 0.2 M to 10 M, 0.2 M to 5 M, 0.2 M to 3 M, 0.2 M to 1 M, 0.2 M to 0.7 M, or 0.2 M to 0.5 M.

In an embodiment, a concentration of Tris-HCl in the buffer of (e) may be 0.001 M to 10 M, for example, 0.001 M to 5 M, 0.001 M to 1 M, 0.001 M to 0.5 M, 0.001 M to 0.1 M, 0.001 M to 0.07 M, 0.005 M to 10 M, 0.005 M to 5 M, 0.005 M to 1 M, 0.005 M to 0.5 M, 0.005 M to 0.1 M, 0.005 M to 0.07 M, 0.01 M to 10 M, 0.01 M to 5 M, 0.01 M to 1 M, 0.01 M to 0.7 M, 0.01 M to 0.5 M, 0.01 M to 0.1 M, 0.01 M to 0.07 M, 0.03 M to 10 M, 0.03 M to 5 M, 0.03 M to 1 M, 0.03 M to 0.7 M, 0.03 M to 0.5 M, 0.03 M to 0.1 M, or 0.03 M to 0.07 M.

When a concentration of the buffer in (e) is too high or too low beyond the above range, a yield or purity of the exosome isolation may decrease.

The buffer in (e) may have a pH of 7.0 to 9.0, but is not limited thereto, and may be, for example, pH 7.5 to pH 9.0, pH 7.5 to pH 8.0, or pH 8.0 to pH 9.0.

The present inventors confirmed that exosomes isolated according to an isolation method of the present disclosure exhibited high efficiency and excellent purity.

Specifically, according to an example of the present disclosure, in order to confirm efficiency of the exosomes, as a result of analyzing electron microscope images and sizes, numbers of particles, and surface charges of the exosomes isolated by different methods and conditions, it was confirmed that the exosomes isolated by the isolation method of the present disclosure exhibited the smallest size in terms of a size, and the highest concentration in terms of a number of particles, and showed a yield about 25 times higher than that of the control group (ultracentrifugation), and about 2.5 to 7.5 times or more higher than comparison groups (Comparative Example 1: using a buffer composition of an existing commercially available PEI-based exosome isolation kit (System Biosciences), Comparative Example 2: using a buffer composition of an existing commercially available PEG + PEI-based exosome isolation kit (Invitrogen)) (See Experimental Example 1).

In addition, in another embodiment of the present disclosure, in order to confirm that the exosome isolation method according to the present disclosure is applicable to samples universally, exosomes were isolated from serum, urine, and cell culture medium of a normal person, and sizes, numbers of particles, and surface charges of the exosomes isolated from each sample were analyzed, and it was confirmed that exosomes were effectively isolated from all samples (see Experimental Example 2).

From the results of the above examples, it was confirmed that the exosomes isolated by the isolation method of the present disclosure may be isolated with high efficiency and high purity, and may be used universally with various samples, and thus, the isolation method of the present disclosure and the exosomes isolated by the method may be widely used in research on diagnosis or treatment methods that require high-purity exosomes.

Another aspect provides a buffer composition for isolating exosomes from a biological sample, including at least one selected from the group consisting of PEG, PEl, and dextran. In regard to the composition, the biological sample, exosomes, and buffers are as described above.

In an embodiment, the buffer may be prepared by adding at least one selected from the group consisting of a PEG solution, a PEI solution, and a dextran solution into physiological saline (NaCl, sodium chloride) or a PBS solution, and the buffer may have a concentration and pH effective for aggregating exosomes from a biological sample. For example, the buffer may have a concentration and a pH of PEG, PEl, or dextran as described above.

Still another aspect provides a kit for isolating exosomes from a biological sample, including a buffer including at least one selected from the group consisting of PEG, PEl, and dextran. In regard to the kit, the biological sample, exosomes, and buffers are as described above. The kit may further include one or more reagents used in the exosome isolation method, and may further include, for example, an elution buffer for neutralization.

Hereinafter, the present disclosure will be described in more detail through examples. However, these examples are intended to illustrate the present disclosure, and the scope of the present disclosure is not limited to these examples.

### [Examples]

### Example 1. Exosome isolation method

Exosomes were isolated by the process shown in FIG. 1. The isolation method is described below by each process.

### 1-1. (a): Deproteinization

In order to remove high molecular weight proteins or aggregated proteins included in the sample, deproteinization was performed. As a sample, human urine or cell culture medium (MSC culture medium) was used. Cellulose, polyvinylidene fluoride (PVDF), and mixed cellulose esters (MCE)-based membranes were used as syringe filters used for deproteinization, and the pore size of the syringe filters was set to 0.8 µm, and then deproteinization was performed.

### 1-2. (b) Exosome aggregation (exosome precipitation)

For the sample deproteinized in (a), in order to aggregate exosomes and to impart charges to the surface, Buffer 1, which is a mixture of various solutions, was prepared and treated. Specifically, Buffer 1 was prepared by adding and mixing a PEG solution (PEG 6000, MW 6,000), a PEI solution, and a dextran solution (dextran sulfate) to a base solution of physiological saline (NaCl, sodium chloride) or PBS to final concentrations of 10 %(w/v), 0.25 %(w/v), 0.15 %(w/v), 0.5 %(w/v), respectively, and then adjusting the final pH to be pH 5.5 ± 0.1. The buffer was treated to the sample deproteinized in (a) to obtain exosome aggregates.

### 1-3. (c): Exosome binding (affinity membrane column)

The aggregate obtained in (b) was added to a column equipped with a membrane which specifically binds to exosomes, and allowed to react at room temperature for 1 minutes to 5 minutes. Polycarbonate or regenerative cellulose, which is friendly to proteins and friendly to positive charges, was used as the material of the membrane.

### 1-4. (d): Centrifugation and washing

The exosomes bound to the membrane through (c) were centrifuged for 1 minute at 500 xg at room temperature or 4 °C and then washed by using PBS or a saline washing buffer. After the washing, centrifugation was performed at 2,500 xg for 10 minutes at room temperature or 4 °C.

### 1-5. (e) Exosome isolation (exosome elusion)

**In order to isolate exosomes by neutralizing Buffer 1 treated in (b), an elution buffer was treated.** Specifically, the elution buffer was prepared by adding and mixing a urea solution, a NaH₂PO₄ solution, and a Tris-HCl pH 5.9 solution to a base solution of PBS or saline to a final concentration of 0.4 M, 0.1 M, and 0.05 M, respectively, and then adjusting the final pH to pH 8.0. The elution buffer was treated to the supernatant obtained in (d) to isolate exosomes through neutralization. After the isolation of exosomes, the final product was obtained by centrifugation at room temperature or 4 °C at 2,500 xg for 5 minutes.

### Experimental Example 1. Comparison of efficiency of isolated exosomes

In order to confirm efficiency of the exosomes isolated by the isolation method of the present disclosure, the size, number of particles, and surface charge of exosomes were analyzed by using a particle size analyzer (ParticleMatrix, Zetaview), and images were taken with an electron microscope (JEM-2010, JEOL, Japan), wherein the exosomes were: exosomes isolated by ultracentrifugation (U/C) (control group); exosomes isolated by the exosome isolation method of the present disclosure (Example 1); and exosomes isolated in the same manner as in Example 1, except that a buffer of a different composition was used instead of Buffer 1 in aggregation of exosomes (Comparative Example 1: use of a buffer composition of an existing commercially available PEI-based exosome isolation kit (System Biosciences), Comparative Example 2: use of a buffer composition of an existing commercially available PEG+PEI-based exosome isolation kit (Invitrogen), Comparative Example 3: use of a PEG-based buffer prepared by adding a PEG solution (PEG 6000) to a base solution of physiological saline or PBS to a final concentration of 10 % (w/v) and adjusting the final pH to 5.5 ± 0.1, and Comparative Example 4: use of a PEG+PEI-based buffer prepared by adding a PEG solution (PEG 6000) and a PEI solution to a base solution of physiological saline or PBS to a final concentration of 5 % (w/v), and 0.5 % (w/v), respectively, and adjusting the final pH to 5.5 ± 0.1).

As a result, as shown in FIGS. 2A and 2B, it was confirmed that the exosomes isolated by the isolation method of the present disclosure exhibited the smallest size in terms of a size, and the highest concentration in terms of a number of particles, and showed a yield about 25 times higher than that of the control group (U/C, ultracentrifugation), about 2.5 times higher than that of Comparative Example 1, which used a buffer of the same composition with an existing commercially available PEI-based exosome isolation kit (System Biosciences), and about 7.5 times higher than that of Comparative Example 2, which used a buffer of the same composition with an existing commercially available PEG+PEI-based exosome isolation kit (Invitrogen), and it was confirmed that the yield was significantly improved compared to Comparative Example 3 which used a PEG-based buffer and Comparative Example 4 which used a different buffer based on PEG + PEI. In addition, as shown in FIG. 2C, the exosomes isolated by the isolation method of the present disclosure exhibited the lowest charge value in terms of a surface charge, and as a result of an electron microscope image analysis, as shown in FIG. 2D, the exosomes isolated by the isolation method according to the present disclosure were confirmed to show the highest purity.

### Experimental Example 2. Confirmation of exosome isolation for each sample

In order to confirm that the exosome isolation method according to the present disclosure may be universally used for samples, after isolating exosomes according to the method of Example 1, by using 10 ml each of serum and urine of a normal person as clinical samples, and 10 ml of MSC culture medium as cell culture medium, the sizes, numbers of particles, and surface charges of the exosomes isolated from each sample were analyzed with a particle size analyzer (ParticleMatrix, Zetaview).

**[Table 1]**

| **Sample** | **Size (nm)** | **Zeta-potential (mV)** | **Particles/ml** |
|---|---|---|---|
| Serum | 103.3 | -34.1 | 9.0 x 10¹⁰ |
| Urine | 111.0 | -27.7 | 5.7 x 10¹⁰ |
| Cell culture media | 120.8 | -24.3 | 1.7 x10¹¹ |

As a result, it was confirmed that exosomes were effectively isolated from all samples, as shown in FIG. 3 and [Table 1].

The above results suggest that the isolation method according to the present disclosure may be used to isolate exosomes from various samples.

### Experimental Example 3. Confirmation of phenotypes of isolated exosomes

Phenotypes of the exosomes isolated by ultracentrifugation (U/C) (control group), and exosomes isolated by the isolation method of the present disclosure (Example 1) were analyzed by flow cytometry (BD, FACSCantoll). As a result, as shown in FIG. 4A, the phenotype of the exosomes isolated by ultracentrifugation did not show any specificity in the phenotype in a non-stained state, but as a result of staining exosome markers, CD63 was confirmed to be expressed. In addition, the phenotype of the exosomes isolated by the isolation method of the present disclosure did not show any specificity in the phenotype in a non-stained state as shown in FIG. 4B, but as a result of staining the exosome markers, CD63 and CD81 were confirmed to be expressed.

### Experimental Example 4. Confirmation of isolation efficiency of exosome isolation buffer for each concentration

Based on the fact that the exosome isolation method of the present disclosure using PEG+PEI+dextran-based Buffer 1 in Experimental Example 1 was confirmed to exhibit an excellent exosome isolation efficiency, the sizes, numbers of particles, and surface charges according to a change in a concentration of Buffer 1 were analyzed by using a particle analyzer (ParticleMatrix, Zetaview).

As a result, as shown in FIGS. 5A to 5C, when a dextran concentration was in a range of 0.01 %(w/v) to 0.4 %(w/v), the exosome size was the smallest, and the highest concentration was exhibited in terms of a number of particles, a significantly high yield was shown, and the lowest charge value was shown in terms of a surface charge. In addition, when compared to Comparative Example 4 which used a PEG+PEI-based buffer, Examples 2 and 3, despite the significantly reduced PEI content compared to Comparative Example 4, were confirmed have significantly improved isolation efficiency by including dextran in a specific content.

### Experimental Example 5. Confirmation of markers of isolated exosomes

Western blot, a protein assay, was performed, in order to confirm protein markers of: exosomes (control group) isolated by ultracentrifugation(U/C); exosomes isolated by the exosome isolation method according to the present disclosure (Example 1); and exosomes isolated in the same manner as in Example 1, except that a buffer of an existing commercially available exosome isolation kit is used instead of Buffer 1 in the aggregation of exosomes (Comparative Example 1: used a buffer composition of an existing commercially available PEI-based exosome isolation kit (System Biosciences), Comparative Example 2: used a buffer composition of an existing commercially available PEG+PEI-based exosome isolation kit (Invitrogen)).

Specifically, the isolated exosomes were dissolved in a radioimmunoprecipitation assay buffer (RIPA). The dissolved proteins were quantified by BCA assay, and 5 µg of the proteins was isolated by using 10 % SDS-PAGE gel and transferred to a 0.22-µm nitrocellulose (NC) membrane. The membrane was incubated in a 5 % skim milk blocking buffer at room temperature for 1 hour and then incubated overnight at 4 °C with primary antibodies (1:1000 dilution, 3 % bovine serum albumin solution). The antibodies used were: anti-CD63 (ab231975, Abcam), anti-CD81 (ab79559, Abcam), anti-CD9 (ab223052, Abcam), and anti-β-actin (A5441, Sigma). After washing with Tris-buffered saline and Tween 20 (TBST), secondary antibodies were added to the membrane for 1 hour at room temperature. The membrane was run with a Western blotting enhanced chemiluminescence (ECL)solution (Supex) to acquire chemiluminescent images, and β-actin was used as an immunoblotting control group.

As a result, as shown in FIG. 6, the exosomes isolated by the isolation method of Example 1 were confirmed to clearly display protein markers of the exosomes compared to the control group or Comparative Examples 1 and 2, and it may be known that exosomes may be isolated with high efficiency and high purity through the isolation method of the present disclosure.

The above description of the present disclosure is for illustrative purposes, and those skilled in the art to which the present disclosure belongs will be able to understand that the examples and embodiments can be easily modified without changing the technical idea or essential features of the disclosure. Therefore, it should be understood that the above examples are not limitative, but illustrative in all aspects.

## Claims

1. A method of isolating exosomes from a biological sample, comprising:
(a) performing deproteinization on a biological sample;
(b) aggregating exosomes by treating the deproteinized sample with a buffer;
(c) binding the aggregated exosomes to a membrane made of a protein-friendly material or a positive charge-friendly material;
(d) centrifuging and washing the exosomes bound to the membrane; and
(e) isolating the washed exosomes through a neutralization reaction.

2. The method of claim 1, wherein the deproteinization of (a) is performed in a syringe filter having a pore size of 0.2 µm to 1.0 µm.

3. The method of claim 1, wherein the buffer used in (b) comprises at least one selected from the group consisting of polyethylene glycol (PEG), polyetherimide (PEI), and dextran.

4. The method of claim 3, wherein a concentration of the PEG is 0.5 % (w/v) to 30 % (w/v).

5. The method of claim 3, wherein a concentration of the PEI is 0.01 % (w/v) to 10 % (w/v).

6. The method of claim 3, wherein a concentration of the dextran is 0.01 % (w/v) to 10 % (w/v).

7. The method of claim 1, wherein the buffer of (b) has a pH of 3.5 to 6.5.

8. The method of claim 1, wherein the protein-friendly material or the positive charge-friendly material in (c) is at least one selected from the group consisting of polycarbonate, anodic aluminum oxide (AAO), cellulose membrane, and regenerative cellulose.

9. The method of claim 1, wherein the neutralization reaction in (e) is performed by treating a buffer comprising at least one selected from the group consisting of urea, monosodium phosphate (NaH₂PO₄), and Tris hydrochloride (Tris-HCl).

10. The method of claim 1, wherein a buffer of (e) has a pH of 7.0 to 9.0.
